# FASCICULE DE BREVET EUROPEEN

(11) **EP 2 553 055 B1**
(45) Date de publication et mention de la délivrance du brevet: **17.12.2014**
(21) Numéro de dépôt: 11717301.3
(22) Date de dépôt: 28.03.2011
(51) Int. Cl.: C10G 70/04, F25J 3/02

(54) **PROCÉDÉ DE TRAITEMENT D'UN COURANT DE GAZ CRAQUÉ ISSU D'UNE INSTALLATION DE PYROLYSE D'HYDROCARBURES ET INSTALLATION ASSOCIÉE**
VERFAHREN ZUR BEHANDLUNG EINES GEKRACKTEN GASSTROMS AUS EINEM KOHLENWASSERSTOFF-PYROLYSE-REAKTOR UND ZUGEHÖRIGE ANLAGE
PROCESS FOR TREATING A STREAM OF CRACKED GAS COMING FROM A HYDROCARBON PYROLYSIS PLANT, AND ASSOCIATED PLANT

(30) Priorité: 29.03.2010 FR 1052271
(43) Date de publication de la demande: 06.02.2013
(73) Titulaire: Technip France, 92400 Courbevoie (FR)
(72) Inventeur: SIMON, Yvon, F-78570 Andresy (FR); LAUGIER, Jean-Paul, F-75013 Paris (FR)
(74) Mandataire: Domenego, Bertrand
(86) Numéro de dépôt international: PCT/FR2011/050671
(87) Numéro de publication internationale: WO 2011/124818

(56) Documents cités:
- WO-A1-2007/018510
- US-A- 4 629 484
- US-A- 5 253 479

## Description

La présente invention concerne un procédé de traitement d'un courant de gaz craqué issu d'une installation de pyrolyse d'hydrocarbures selon le préambule de la revendication 1

Le gaz craqué est obtenu à partir d'une installation de pyrolyse d'hydrocarbures, telle qu'un four de vapocraquage. Le gaz introduit dans l'installation de pyrolyse contient avantageusement de l'éthane, du propane, du butane, du naphta et/ou du gasoil seul ou en mélange.

Le procédé du type précité est destiné à traiter le gaz craqué pour pouvoir extraire plus de 99,5% en moles de l'éthylène contenu dans le gaz craqué, et pour obtenir une coupe riche en éthylène présentant une teneur supérieure à 99,95% en moles d'éthylène.

Un procédé du type précité qui permet d'obtenir de telles performances est décrit par exemple dans US-5 253 479.

Ce procédé est mis en oeuvre pour traiter de très grands volumes de gaz craqué, par exemple supérieurs à 50 tonnes par heure, notamment supérieurs à 100 tonnes par heure.

Pour garantir à la fois une très grande pureté du courant d'éthylène produit et un taux de récupération d'éthylène maximal, il est nécessaire de refroidir le gaz craqué par étape jusqu'à des températures inférieures à - 100°C et notamment inférieures à - 120°C.

A cet effet, le gaz craqué est refroidi successivement dans des régions d'échange thermique de plus en plus froides. Le gaz craqué est condensé partiellement dans chaque région d'échange thermique.

A la sortie de chaque région d'échange thermique, le liquide condensé contenant les hydrocarbures en C₂⁺ est récupéré.

Les liquides condensés à plus haute température sont envoyés dans une colonne amont de déméthanisation pour récupérer en pied une première coupe riche en hydrocarbures en C₂⁺.

Les liquides intermédiaires et aval obtenus à plus basses températures sont envoyés dans une colonne intermédiaire de déméthanisation qui produit en pied une deuxième coupe riche en hydrocarbures en C₂⁺.

Le courant de tête issu de la colonne amont est introduit dans la colonne intermédiaire.

Dans US-5 253 479, pour améliorer encore la récupération d'éthylène, le courant de tête issu de la colonne intermédiaire de déméthanisation est introduit, après refroidissement, dans une troisième colonne de séparation. Le pied de la troisième colonne est alors réintroduit partiellement, après pompage dans une première pompe cryogénique, en reflux dans la colonne intermédiaire. La tête de la troisième colonne est introduite, après refroidissement et pompage par une deuxième pompe cryogénique, dans un absorbeur d'éthylène qui constitue la quatrième colonne de distillation.

Le procédé décrit dans US-5 253 479 est donc particulièrement efficace pour obtenir une excellente récupération de l'éthylène.

Compte tenu de la présence de quatre colonnes de distillation, et de deux pompes cryogéniques, la structure de l'installation et la consommation énergétique du procédé peuvent néanmoins encore être améliorées.

US-4 629 484 décrit un autre procédé du type précité.

Un but de l'invention est donc d'obtenir, avec un investissement minimal, et une simplification des équipements, un procédé de traitement d'un gaz craqué qui permet d'extraire quasiment la totalité de l'éthylène contenu dans le gaz craqué, tout en présentant des performances énergétiques et opératoires améliorées.

A cet effet, l'invention a pour objet un procédé selon la revendication 1.

Le procédé selon l'invention peut comprendre l'une ou plusieurs des caractéristiques des revendications 2 à 13, prise(s) isolément ou suivant toute(s) combinaison(s) techniquement possible(s).

L'invention a en outre pour objet une installation selon la revendavtion 14.

L'installation selon l'invention peut comprendre l'une ou plusieurs des caractéristiques des revendications 15 à 17, prise(s) isolément ou suivant toute(s) combinaison(s) techniquement possible(s).

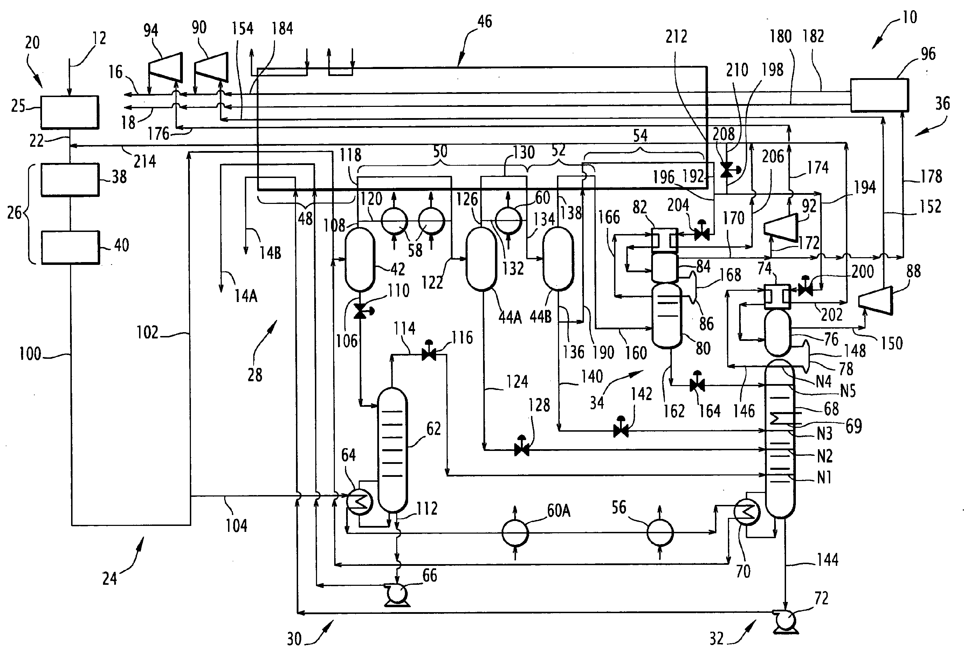

L'invention sera mieux comprise à la lecture de la description qui va suivre, donnée uniquement à titre d'exemple, et faite en se référant au dessin annexé, sur lequel :
- la figure unique est un schéma synoptique fonctionnel d'une première installation de traitement selon l'invention, destinée à la mise en oeuvre d'un premier procédé selon l'invention.

Dans tout ce qui suit, une même référence désigne un courant circulant dans une conduite et la conduite qui transporte ce courant. Par ailleurs, sauf indication contraire, les pourcentages sont des pourcentages molaires et les pressions s'entendent en bars relatifs.

Une première unité 10 de vapocraquage selon l'invention est représentée sur la figure unique.

Cette unité 10 produit, à partir d'une charge 12, des courants 14A, 14B riches en C₂⁺ destinés à former une coupe riche en éthylène, un gaz combustible 16 et un courant 18 riche en hydrogène.

L'unité 10 comprend une installation 20 de pyrolyse d'hydrocarbures produisant un gaz craqué brut 22 et une installation 24 de traitement du gaz craqué brut 22.

L'installation de pyrolyse 20 comporte plusieurs fours de vapocraquage 25. Les fours de vapocraquage 25 sont propres à faire circuler la charge 12 pour la chauffer à une température supérieure à 800°C. Ceci provoque le craquage thermique des molécules d'hydrocarbures contenues dans la charge 12 afin de former le gaz craqué brut 22.

L'installation de fractionnement 24 comporte successivement un ensemble de compression 26 et un ensemble 28 de refroidissement et de séparation successive du gaz craqué.

L'installation 24 comprend en outre un ensemble amont de distillation 30, un ensemble intermédiaire de distillation 32 et un ensemble additionnel 34 d'absorption d'éthylène. L'installation 24 comprend également un ensemble aval 36 de détente et de réchauffage du gaz combustible.

L'ensemble de refroidissement et de compression 26 comprend un appareil de refroidissement (non représenté), un compresseur primaire 38 et un compresseur secondaire 40, le compresseur secondaire 40 étant situé en aval du compresseur primaire 38.

L'ensemble de refroidissement et de séparation 28 comprend un ballon séparateur amont 42, un premier ballon séparateur intermédiaire 44A, un deuxième ballon séparateur intermédiaire 44B. Il comprend en outre un échangeur thermique intégré comportant une boîte froide 46. La boîte froide 46 comprend une région amont 48 d'échange thermique, une région intermédiaire 50 d'échange thermique, une région aval 52 d'échange thermique et une région additionnelle 54 d'échange thermique, de plus en plus froides.

L'ensemble de refroidissement 28 comprend en outre un cycle de réfrigération au propane ou au propylène présentant un échangeur amont 56 de cycle et un cycle de réfrigération à l'éthylène comprenant un premier ensemble d'échangeurs intermédiaires 58 de cycle et un deuxième ensemble d'échangeurs intermédiaires de cycle 60. Optionnellement, l'ensemble de refroidissement 28 comporte en outre un échangeur amont 60A de refroidissement additionnel alimenté par un fluide réfrigérant formé par un courant de rebouillage intermédiaire d'un séparateur d'hydrocarbures en C₂ ou par un courant d'éthane destiné à être vaporisé.

L'ensemble de distillation amont 30 comporte une colonne amont 62 de distillation, un rebouilleur amont 64 de fond de colonne et une pompe amont 66 de fond de colonne.

La colonne 62 opère une pression comprise entre 10 bars et 14 bars. Elle comporte par exemple entre 14 et 20 plateaux théoriques.

L'ensemble intermédiaire de distillation 32 comporte une colonne intermédiaire 68 de déméthanisation, un échangeur intégré 69, un échangeur intermédiaire 70 de rebouillage et une pompe intermédiaire 72 de fond de colonne.

L'ensemble intermédiaire 32 comporte en outre un premier échangeur thermique de reflux 74 et un premier ballon séparateur de reflux 76 raccordés à la colonne 68 par une conduite 78 de descente de liquide, conformée pour empêcher le passage de gaz depuis la colonne 62 vers le ballon 76. Cet ensemble 32 évite l'installation de pompes cryogéniques de reflux.

La colonne 68 opère à une pression comprise entre 10 bars et 14 bars. Elle comporte par exemple entre 22 et 28 plateaux théoriques.

L'échangeur intégré 69 est placé dans la colonne 68 entre une partie supérieure et une partie inférieure de la colonne 69.

L'ensemble 34 de distillation aval comprend une colonne 80 d'absorption d'éthylène, un deuxième échangeur thermique de reflux 82 et un deuxième ballon séparateur 84 de reflux raccordé à la colonne d'absorption d'éthylène 80 par une conduite 86 de descente de liquide, conformée pour empêcher le passage de gaz depuis la colonne 80 vers le ballon 84. Cet ensemble 34 évite l'installation de pompes cryogénique de reflux.

Comme on le verra plus bas, la pompe cryogénique de reflux présente dans US 5,253,479 peut être supprimée de la colonne 68, ce qui conduit à la production d'un liquide nécessaire à l'absorption d'éthylène dans l'ensemble 34 au moyen de l'échangeur 82.

La colonne 80 opère à une pression comprise entre 30 bars et 40 bars. Elle comporte par exemple entre 4 et 8 plateaux théoriques.

L'ensemble aval 36 comprend une première turbine de détente dynamique 88 accouplée à un premier compresseur 90, une deuxième turbine de détente dynamique 92 accouplée à un deuxième compresseur 94 et une unité 96 de purification d'hydrogène.

Un premier procédé selon l'invention, mis en oeuvre dans l'unité 10 va maintenant être décrit.

Initialement, la charge 12 comporte de l'éthane, du propane, du butane, du naphta et/ou du gasoil seul ou en mélange. Elle est introduite dans les fours de vapocraquage 25 pour être chauffée à une température supérieure à 800°C et subir un craquage thermique.

Un gaz craqué brut est extrait des fours à une température supérieure à 800°C, puis est refroidi rapidement pour engendrer le flux 22 à une température supérieure à 160°C et à une pression supérieure à 1 bar.

Le gaz 22 est ensuite refroidi et est introduit dans le compresseur primaire 38 pour y être comprimé à une pression supérieure à 10 bars, puis dans le compresseur secondaire 40 pour y être comprimé à une pression supérieure à 30 bars. Le courant de gaz craqué brut comprimé 100 est ensuite séparé en une première fraction 102 de gaz craqué brut et en une deuxième fraction 104 de gaz craqué brut.

La première fraction de gaz craqué brut 102 est convoyée jusqu'à la boîte froide 46 pour y être refroidie jusqu'à une température inférieure à - 25°C et notamment comprise entre - 30°C et - 40°C pour y être partiellement condensée dans la région amont 48 d'échange thermique.

La deuxième fraction de gaz craqué brut 104 est refroidie successivement dans l'échangeur amont de rebouillage 64, dans l'échangeur optionnel 60A de refroidissement, dans l'échangeur amont de cycle au propane ou propylène 56, avant d'être refroidie à nouveau dans l'échangeur intermédiaire de rebouillage 70 pour atteindre une température inférieure à - 25°C et notamment comprise entre - 30°C et - 40°C. La fraction 104 est en outre partiellement condensée.

La température du courant 104 en amont de l'échangeur 64 est comprise entre - 2°C et - 12°C et la température du courant 104 en aval du rebouillage dans l'échangeur 70 est comprise entre - 30°C et - 40°C.

Le rapport du débit molaire de la première fraction 102 au débit molaire de la deuxième fraction 104 est par exemple compris entre 0,25 et 0,40.

La première fraction 102 et la deuxième fraction 104 formant un courant de gaz craqué partiellement condensé sont ensuite introduites dans le ballon séparateur amont 42 pour y être séparées en un liquide amont 106 et en un courant amont gazeux 108 de gaz craqué.

Le liquide amont 106 contient entre 45% et 55% en moles des hydrocarbures en C₂ présents dans le gaz craqué brut 22 et entre 85 % et 95% en moles des hydrocarbures en C₃⁺ contenus dans le gaz craqué brut 22.

Le liquide amont 106 est ensuite détendu dans une première vanne de détente statique 110 pour être introduit à un niveau supérieur de la colonne amont de déméthanisation 62.

La colonne amont 62 produit, en pied, un premier courant liquide 112 riche en hydrocarbures en C₂⁺ qui est convoyé jusqu'à la pompe amont 66 pour produire un premier courant riche 14A en hydrocarbures en C₂⁺ qui est pompé. Le premier courant 14A est destiné à être convoyé vers une colonne de dééthanisation pour en extraire la coupe riche en éthylène qui sera ensuite purifiée pour atteindre une teneur en éthylène supérieure à 99,95%.

La teneur molaire en hydrocarbures en C₂ dans le premier courant 112 est supérieure à 50 %. La teneur molaire en méthane dans le premier courant 112 est inférieure à 0,01 %.

La colonne amont 62 produit en outre un courant de tête gazeux 114 riche en méthane. Le courant de tête gazeux 114 est introduit dans la colonne intermédiaire 68 à un niveau inférieur N₁ de cette colonne, après passage dans la vanne 116.

Une première partie 118 du courant amont de gaz craqué 108 est ensuite introduite dans la région intermédiaire 50 d'échange thermique pour y être refroidie jusqu'à une température inférieure à - 60°C et notamment comprise entre - 65°C et - 76°C et pour y être partiellement condensée.

Une deuxième partie 120 du courant amont de gaz craqué 108 est introduite successivement dans le premier ensemble d'échangeurs intermédiaires 58 du cycle à l'éthylène pour y être refroidie jusqu'à une température inférieure à - 60°C et par exemple comprise entre - 65°C et - 76°C, et pour y être partiellement condensée.

Le rapport du débit de la première partie 118 au débit de la deuxième partie 120 est par exemple compris entre 0,15 et 0,25.

La première partie 118 et la deuxième partie 120 sont ensuite mélangées pour former un courant amont de gaz craqué partiellement condensé 122 qui est introduit dans le premier ballon séparateur intermédiaire 44A. La fraction molaire de liquide dans le courant amont de gaz craqué partiellement condensé 122 est supérieure à 25%.

Le courant 122 est ensuite séparé dans le premier ballon intermédiaire 44A en un premier liquide intermédiaire 124 et en un premier courant intermédiaire de gaz craqué 126.

Le premier liquide intermédiaire 124 est détendu dans une troisième vanne de détente statique 128 jusqu'à une pression inférieure à 14 bars avant d'être introduit à un niveau N₂ de la colonne intermédiaire situé au-dessus du niveau N₁.

Le premier liquide intermédiaire 124 comporte entre 60% et 75% des hydrocarbures en C₂ contenus dans le gaz craqué brut 22 et entre 10% en moles et 15% en moles des hydrocarbures en C₃⁺ contenus dans le gaz craqué brut 22.

Une première partie 130 du premier courant intermédiaire de gaz craqué 126 est ensuite introduite dans la région intermédiaire 50 d'échange thermique pour y être refroidie à une température inférieure à - 90°C et notamment comprise entre - 92°C et - 99°C et pour y être partiellement condensée.

Une deuxième partie 132 du courant intermédiaire 126 est introduite dans le deuxième ensemble d'échangeur intermédiaire 60 du cycle de réfrigération à l'éthylène pour y être refroidie à une température inférieure à - 90°C et notamment comprise entre -92°C et - 99°C et pour y être partiellement condensée.

Les premières parties 130 et 132 sont ensuite mélangées pour former un premier courant intermédiaire 134 de gaz craqué partiellement condensé. La teneur molaire en liquide du courant 134 est supérieure à 15 %.

Le courant 134 est ensuite introduit dans le deuxième ballon séparateur intermédiaire 44B pour y être séparé en un deuxième liquide intermédiaire 136 et en un deuxième courant intermédiaire 138 de gaz craqué.

Le deuxième liquide intermédiaire 136 contient entre 55% molaire et 65 % molaires des hydrocarbures en C₂ contenus dans le courant de gaz craqué brut 22 et entre 0,5% molaires et 1,5% molaire des hydrocarbures en C₃⁺ contenus dans le gaz craqué brut 22.

Une première partie 140 du deuxième liquide intermédiaire 136 est ensuite détendue dans une quatrième vanne de détente statique 142 jusqu'à une pression inférieure à 14 bars pour être introduite à un niveau N₃ de la colonne intermédiaire 68 situé au-dessus du niveau N₂.
· Le niveau N₃ est situé au dessous de l'échangeur intégré 69.

La colonne intermédiaire de déméthanisation 68 produit, en pied, un deuxième courant de fond 144 riche en hydrocarbures en C₂⁺. Le courant 144 est pompé à travers la pompe intermédiaire 72 jusqu'à une pression supérieure à 20 bars pour former un deuxième courant 14B riche en C₂⁺ destiné à être envoyé vers la colonne de dééthanisation.

La teneur molaire en hydrocarbures en C₂⁺ dans le courant 144 et dans le courant 14B est supérieure à 90%. La teneur molaire en méthane dans les courants 144, 14B est inférieure à 0,01 % molaire.

La colonne intermédiaire 68 produit, en tête, un courant intermédiaire de tête 146 qui est refroidi et partiellement condensé jusqu'à une température inférieure à - 115°C, par exemple comprise entre - 118°C et - 123°C, dans le premier échangeur thermique de reflux 74.

Le courant intermédiaire de tête partiellement condensé est ensuite introduit dans le premier ballon de reflux 76 pour y être séparé en un premier courant de reflux liquide 148 et en un premier courant de gaz combustible à haute pression 150.

Le premier courant de reflux 148 est introduit à un niveau de tête N₄ de la colonne 68 à travers la conduite 78. Le niveau N₄ est situé au-dessus de l'échangeur intégré 69. Ce système à reflux à écoulement gravitaire évite l'installation d'une pompe cryogénique.

Le premier courant de gaz combustible 150 contient plus de 90% en moles de méthane et moins de 0,1% en moles d'hydrocarbures en C₂. Le courant 150 est ensuite avantageusement détendu dans la première turbine de détente dynamique 88 jusqu'à une pression par exemple inférieure à 4,5 bars pour former un courant 152 de combustible à basse pression refroidi à une température inférieure à -135°C.

Le courant 152 est alors introduit dans la boîte froide 46 pour y être réchauffé successivement dans la région additionnelle 54, dans la région aval 52, dans la région intermédiaire 50 et dans la région amont 48 d'échange thermique, par échange thermique avec les courants circulant respectivement dans ces régions.

Le premier courant de combustible réchauffé 154 issu de la première région d'échange thermique 48 est ensuite amené dans le premier compresseur 90 couplé à la turbine 88 pour être comprimé jusqu'à une pression supérieure à 5,0 bars et former une partie du courant de gaz combustible 16.

Dans une variante, l'ensemble formé par la turbine de détente 88 et le compresseur 90 est remplacé par une vanne de détente statique globale.

Le deuxième courant intermédiaire 138 de gaz craqué issu du deuxième ballon intermédiaire 44B est ensuite refroidi et partiellement condensé dans la région aval d'échange thermique 52 de la boîte froide 46 pour former un courant intermédiaire 160 de gaz craqué partiellement condensé.

Le courant 160 est ensuite introduit à un niveau inférieur de la colonne d'absorption d'éthylène 80 à une température inférieure à - 110°C et notamment comprise entre - 115°C et - 120°C.

La colonne d'absorption d'éthylène 80 opère à une pression par exemple comprise entre 30 bars et 36 bars.

Le courant de pied additionnel 162 produit au pied de la colonne 80 est ensuite détendu jusqu'à une pression inférieure à 15 bars à travers une cinquième vanne de détente statique 164 avant d'être introduit à un niveau N₅ de la colonne intermédiaire 68 de déméthanisation situé entre le niveau N₃ et le niveau N₄.

Ce courant 162 contient entre 3,0 % et 5,0 % en moles des hydrocarbures en C₂ présents dans le gaz craqué 22 et entre 0,01 % et 0,04 % en moles des hydrocarbures en C₃⁺ présents dans le gaz craqué brut 22.

Le courant de tête 166 additionnel produit dans la colonne 80 est introduit dans le deuxième échangeur thermique de reflux 82 pour être refroidi à une température inférieure à - 115°C et notamment comprise entre - 118°C et - 130°C et être partiellement condensé.

Le courant de tête additionnel 166 partiellement condensé est ensuite introduit dans le deuxième ballon séparateur de reflux 84 pour former un deuxième courant de reflux liquide 168 et un courant gazeux traité 170 à haute pression.

Le deuxième courant de reflux liquide 168 est introduit en reflux dans la colonne d'absorption d'éthylène 80 à travers la conduite 86. Ce système d'absorption à reflux à écoulement gravitaire évite l'installation d'une pompe cryogénique.

Une première partie 172 du courant traité 170 est envoyée vers la deuxième turbine de détente dynamique 92 pour y être détendue à une pression inférieure à 4,5 bars et former un deuxième courant de gaz combustible 174 basse pression refroidi à une température inférieure à -140 °C.

Le courant 174 est ensuite réchauffé successivement à travers la région additionnelle 54, la région aval 52, la région intermédiaire 50 et la région amont 48 d'échange thermique au sein de la boîte froide 46 pour former un deuxième courant de gaz combustible réchauffé 176.

Le deuxième courant 176 est ensuite introduit dans le deuxième compresseur 94 pour former une partie du courant de gaz combustible 16.

Dans une variante, l'ensemble turbine de détente 92 et le compresseur 94 est remplacé par une vanne de détente statique globale.

Une deuxième partie 178 du courant gazeux traité 170 est convoyée jusqu'à l'unité de purification cryogénique d'hydrogène 96 pour former un courant 180 riche en hydrogène et un troisième courant de combustible basse pression 182.

La teneur en hydrogène dans le courant riche en hydrogène 180 produit par l'unité 96 est supérieure à 90% molaires. La température du courant 180 est inférieure à -125 °C.

Le courant 180 est ensuite passé successivement dans la région additionnelle d'échange thermique 54, dans la région aval d'échange thermique 52, dans la région intermédiaire d'échange thermique 50 et dans la région amont d'échange thermique 48 de la boîte froide 46 pour y être réchauffé par échange thermique avec les courants circulant respectivement dans ces régions et former le courant riche en hydrogène 18.

Le troisième courant de combustible 182 issu de l'unité 96 présente une température inférieure à -125°C. Ce courant 182 est alors passé successivement dans la région additionnelle d'échange thermique 54, dans la région aval d'échange thermique 52, dans la région intermédiaire d'échange thermique 50, puis dans la région amont d'échange thermique 48 pour se réchauffer par échange thermique avec les courants circulant respectivement dans ces régions.

Le troisième courant de combustible réchauffé 184 est ensuite mélangé au premier courant de combustible réchauffé 154, après sa compression dans le compresseur 94 et au deuxième courant de combustible réchauffé 176 pour former une partie du courant de combustible 16.

Selon l'invention, les frigories nécessaires à la condensation du courant de tête gazeux intermédiaire 146 issu de la colonne intermédiaire 68 sont fournies par échange thermique avec un courant circulant dans un cycle semi-ouvert de type JOULE-THOMPSON.

Dans une variante (non représentée), les frigories nécessaires à la condensation du courant de tête gazeux intermédiaire 146 issu de la colonne intermédiaire 68 sont fournies partiellement dans le même échangeur 74 par échange thermique soit avec le courant de combustible basse pression 152 soit avec le deuxième courant de gaz combustible 174, obtenus respectivement à l'échappement de la turbine de détente 88 ou à l'échappement de la turbine de détente 92, en complément du cycle semi-ouvert de type JOULE-THOMPSON.

En outre, les frigories nécessaires à la condensation du courant de tête 166 issu de la colonne d'absorption 80 sont fournies par échange thermique avec un courant circulant dans un cycle semi-ouvert de type JOULE-THOMPSON.

Dans une variante (non représentée), les frigories nécessaires à la condensation du courant de tête 166 issu de la colonne d'absorption 80 sont fournies partiellement par échange thermique dans le même échangeur 82 soit avec le courant de combustible basse pression 152 soit avec le deuxième courant de gaz combustible 174, obtenus respectivement à l'échappement de la turbine de détente 88 ou à l'échappement de la turbine de détente 92, en complément du cycle semi-ouvert de type JOULE-THOMPSON.

A cet effet, une deuxième partie 190 du deuxième liquide intermédiaire 136 issu du ballon séparateur 44B est prélevée et est refroidie dans la région additionnelle 54 d'échange thermique jusqu'à une température inférieure à -125°C, pour former une deuxième partie prélevée refroidie 192.

La deuxième partie prélevée refroidie 192 est ensuite séparée en une première fraction 194 de refroidissement du premier échangeur thermique de reflux 74, en une deuxième fraction 196 de refroidissement du deuxième échangeur thermique de reflux 82 et en une troisième fraction 198 de refroidissement d'appoint.

A cet effet, la première fraction 194 est détendue dans une sixième vanne de détente statique 200 jusqu'à une pression inférieure à 2 bars ce qui provoque son refroidissement jusqu'à une température à -140°C. Elle est ensuite réchauffée par échange thermique avec le courant de tête 146 dans le premier échangeur thermique 74 jusqu'à une température supérieure à -120°C, pour former une première fraction réchauffée 202.

De même, la deuxième fraction 196 est détendue dans une septième vanne 204 de détente statique jusqu'à une pression inférieure à 2 bars, ce qui abaisse sa température en dessous de -140°C. La deuxième fraction détendue est ensuite réchauffée par échange thermique avec le courant de tête 166 dans le deuxième échangeur thermique de reflux 82 jusqu'à une température supérieure à -130 °C pour former une deuxième fraction réchauffée 206.

La troisième fraction 198 est détendue dans une huitième vanne de détente statique 208 jusqu'à une pression inférieure à 2 bars pour produire une troisième fraction détendue 210 à une température inférieure à -140°C.

Les fractions détendues 202, 206 et 210 sont ensuite mélangées pour former un courant 212. Le courant 212 est réchauffé successivement dans la région additionnelle d'échange thermique 54, dans la région aval d'échange thermique 52, dans la région intermédiaire d'échange thermique 50 et dans la région amont d'échange thermique 48 par échange thermique avec les courants circulant respectivement dans ces régions pour former un courant réchauffé 214. Le courant 214 est alors réintroduit en mélange dans le gaz craqué 22 en amont du premier compresseur 38 pour être recomprimé dans les compresseurs 38, 40.

Le prélèvement d'une partie d'un liquide intermédiaire 136 et sa détente dans des vannes statiques 200, 204, 208 produit donc les frigories nécessaires pour former les courants de reflux des colonnes 68 et 80 et permet de boucler le bilan thermique sur la boîte froide 46, en formant un cycle mixte de type JOULE-THOMPSON intégrant l'ensemble de compression 26 des gaz craqués.

Cette disposition particulière du procédé de traitement, et de l'installation de traitement 24 associée, simplifie grandement la structure de l'installation 24 en évitant d'avoir à pomper des fluides cryogéniques et en réalisant une meilleure intégration thermique.

En particulier, le courant de tête gazeux intermédiaire 146 n'est pas pompé vers la colonne d'absorption 80, mais est redirigé vers les régions d'échange thermique 54 à 48, ce qui permet d'éviter de relier la colonne 68 à la colonne 80 par une pompe cryogénique.

De même, l'échangeur intégré 69 disposé dans la colonne 68 permet d'utiliser une seule colonne de distillation, et donc de réduire le nombre d'équipements dans l'installation.

De plus, les colonnes de déméthanisation 62, 68 produisant des courants de pied 14A, 14B respectifs qui présentent des compositions différentes, le fractionnement ultérieur dans le dééthaniseur est amélioré par une diminution de la consommation énergétique.

En tout état de cause, l'installation de traitement 24, et le procédé mis en oeuvre dans cette installation 24, permettent de récupérer plus de 99,5% en moles de l'éthylène présent dans le gaz craqué 22, avec une consommation énergétique diminuée et une installation de structure simplifiée, qui présente un coût réduit de mise en place et de maintenance.

En outre, le refroidissement d'une fraction 104 du courant de gaz craqué brut comprimé 100 dans les échangeurs thermiques de rebouillage 64, 70 des colonnes 62, 68 minimise l'énergie nécessaire à ce refroidissement et le nombre d'équipements, puisque le même échangeur thermique 64, 70 assure la fonction de rebouillage d'une colonne 62, 68 et de refroidissement du gaz craqué brut.

Le gaz riche en hydrogène 170 issu de la colonne d'absorption d'éthylène 80 est en outre avantageusement traité pour récupérer un courant riche en hydrogène 18 en permettant à l'excès de gaz traité 170 riche en hydrogène issu de la colonne 80 d'être éventuellement détendu à travers une turbine 92 pour générer un produit froid pouvant être réchauffé dans la boîte froide 46.

Dans une variante, au moins deux parmi la région amont d'échange thermique 48, la région intermédiaire d'échange thermique 50, la région aval d'échange thermique 52 et la région additionnelle d'échange thermique 54 sont disposées dans des échangeurs thermiques séparés, qui ne sont pas intégrés au sein de la boîte froide 46.

Dans une variante, chacune des régions 48, 50, 52, 54 est disposée dans un échangeur thermique propre.

## Revendications

1. Procédé de traitement d'un courant de gaz craqué (22) issu d'une installation (20) de pyrolyse d'hydrocarbures, du type comprenant les étapes suivantes :
- refroidissement amont et condensation partielle d'un courant de gaz craqué brut (100) dans au moins une région amont (48) d'échange thermique ;
- séparation du courant de gaz craqué brut partiellement condensé dans au moins un séparateur amont (42) pour récupérer un liquide amont (106) et un courant amont (108) de gaz craqué ;
- introduction du liquide amont (106) dans une colonne amont (62) de déméthanisation pour récupérer en tête de la colonne amont (62), un courant amont de tête (114) riche en méthane et, en pied de la colonne amont, un premier courant liquide (112) riche en hydrocarbures en C₂⁺ ;
- refroidissement intermédiaire et condensation partielle du courant amont de gaz craqué (108) dans au moins une région intermédiaire (50) d'échange thermique ;
- séparation du courant amont de gaz craqué partiellement condensé dans au moins un séparateur intermédiaire (44A, 44B) pour récupérer au moins un liquide intermédiaire (124, 136) et un courant intermédiaire (138) de gaz craqué ;
- introduction du ou de chaque liquide intermédiaire (124, 140) dans une colonne intermédiaire (68) de déméthanisation pour récupérer, en tête de la colonne intermédiaire (68), un courant intermédiaire de tête (146), et en pied de la colonne intermédiaire (68), un deuxième courant liquide (144) riche en hydrocarbures en C₂⁺ ;
- introduction d'au moins une partie du courant amont de tête (114) issu de la colonne amont (62) dans la colonne intermédiaire (68) ;
- refroidissement aval et condensation partielle du courant intermédiaire de gaz craqué (138) dans au moins une région aval (52) d'échange thermique ;
- séparation du courant intermédiaire de gaz craqué partiellement condensé (160) dans un ensemble aval de séparation (80) pour récupérer un liquide aval (162) et un courant aval de gaz traité (170) ;
- introduction du liquide aval (162) dans la colonne intermédiaire de déméthanisation (68) ;
**caractérisé en ce que** le procédé comprend les étapes suivantes :
- prélèvement d'une partie d'un liquide intermédiaire (136) issu d'un séparateur intermédiaire (44A, 44B) et de refroidissement de la partie prélevée (190) dans une région additionnelle (54) d'échange thermique ;
- détente d'au moins une première fraction (194) de refroidissement obtenue à partir de la partie prélevée (190) et mise en relation d'échange thermique de la première fraction de refroidissement détendue avec le courant intermédiaire de tête (146) dans un premier échangeur thermique de tête (74) pour condenser au moins partiellement le courant intermédiaire de tête (146) ;
- séparation du courant intermédiaire de tête partiellement condensé dans un premier séparateur (76) de reflux pour former un courant liquide (148) de reflux introduit dans la colonne intermédiaire (68) par écoulement gravitaire, et un premier courant gazeux de combustible (150) ;
- détente et réchauffage du premier courant gazeux de combustible (150) par passage dans au moins une région parmi la région additionnelle (54) d'échange thermique, la région aval (52) d'échange thermique, la région intermédiaire (50) d'échange thermique, et la région amont (48) d'échange thermique.

2. Procédé selon la revendication 1, **caractérisé en ce que** la colonne intermédiaire (68) comporte un échangeur thermique intégré (69), le ou chaque liquide intermédiaire (124, 140) étant introduit au dessous de l'échangeur thermique intégré (69), le liquide aval (162) étant introduit au-dessus de l'échangeur thermique intégré (69).

3. Procédé selon la revendication 1 ou 2, **caractérisé en ce qu'**il comprend une étape de formation du courant de gaz craqué brut (100) par compression d'un gaz craqué (22) issu de l'installation de pyrolyse (20) dans au moins un appareil de compression, le procédé comprenant les étapes suivantes :
- réchauffage de la première fraction de refroidissement (194) dans le premier échangeur thermique de tête (74) dans au moins une parmi la région additionnelle (54) d'échange thermique, la région aval (52) d'échange thermique, la région intermédiaire (50) d'échange thermique et la région amont (48) d'échange thermique ; et
- introduction de la première fraction de refroidissement réchauffée dans le gaz craqué (22) en amont ou au sein de l'appareil de compression (38, 40).

4. Procédé selon l'une quelconque des revendications précédentes, **caractérisé en ce que** le premier courant gazeux de combustible (150) issu du premier séparateur de reflux (76) est détendu dans une première turbine de détente dynamique (88), puis est réchauffé dans au moins une parmi la région additionnelle (54) d'échange thermique, la région aval (52) d'échange thermique, la région intermédiaire (50) d'échange thermique, et la région amont (48) d'échange thermique, le procédé comprenant une étape de recompression du premier courant de combustible réchauffé (154) dans au moins un premier compresseur (90) attelé à la première turbine de détente dynamique (88).

5. Procédé selon l'une quelconque des revendications précédentes, **caractérisé en ce que** le premier courant gazeux de combustible (150) issu du premier séparateur de reflux (76) est détendu dans une vanne de détente statique.

6. Procédé selon l'une quelconque des revendications précédentes, **caractérisé en ce que** l'ensemble aval de séparation comporte une colonne (80) d'absorption d'éthylène, le procédé comprenant les étapes suivantes :
- introduction du courant intermédiaire de gaz craqué partiellement condensé (160) dans la colonne d'absorption d'éthylène (80),
- récupération d'un courant additionnel (166) de tête gazeux issu de la colonne d'absorption d'éthylène (80) ;
- détente d'une deuxième fraction de refroidissement (196) obtenue à partir de la partie prélevée (190) dans le liquide intermédiaire, et
- mise en relation d'échange thermique de la deuxième fraction de refroidissement détendue avec le courant additionnel de tête (166) dans un deuxième échangeur thermique de tête (82) pour condenser au moins partiellement le courant additionnel de tête (166) ;
- introduction du courant additionnel de tête partiellement condensé dans un deuxième séparateur de reflux (84) pour former un deuxième courant liquide de reflux (168) introduit dans la colonne d'absorption d'éthylène (80) par écoulement gravitaire et un courant gazeux traité (170).

7. Procédé selon la revendication 6, **caractérisé en ce qu'**il comprend les étapes suivantes :
- formation du courant de gaz craqué brut (100) par compression d'un gaz craqué (22) issu de l'installation de pyrolyse (20) dans un appareil de compression (38, 40) :
- réchauffage de la deuxième fraction de refroidissement (190), en aval du deuxième échangeur thermique de tête (82), dans au moins une parmi la région additionnelle (54) d'échange thermique, la région aval (52) d'échange thermique, la région intermédiaire (50) d'échange thermique, et la région amont (48) d'échange thermique ;
- introduction de la deuxième fraction de refroidissement réchauffée dans le gaz craqué (22) issu de l'installation de pyrolyse (20), en amont ou au sein de l'appareil de compression (38, 40).

8. Procédé selon l'une quelconque des revendications 6 ou 7, **caractérisé en ce qu'**il comprend les étapes suivantes :
- détente d'au moins une première partie (172) du courant gazeux traité (170) dans au moins une deuxième turbine de détente dynamique (92),
- réchauffage après détente de la première partie (174) du courant gazeux traité (170) dans au moins une parmi la région additionnelle (54) d'échange thermique, la région aval (52) d'échange thermique, la région intermédiaire (50) d'échange thermique, et la région amont (48) d'échange thermique ;
- compression de la première partie (172) réchauffée dans au moins un deuxième compresseur (94) accouplé à la deuxième turbine de détente dynamique (92).

9. Procédé selon l'une quelconque des revendications 6 ou 7, **caractérisé en ce qu'**il comprend une étape de détente d'au moins une première partie (172) du courant gazeux traité (170) dans une vanne de détente statique.

10. Procédé selon l'une quelconque des revendications 6 à 9, **caractérisé en ce qu'**au moins une deuxième partie (178) du courant gazeux traité (170) est introduite dans une unité (96) de purification d'hydrogène pour produire un courant riche en hydrogène (180) et un courant auxiliaire (182) de gaz combustible, et éventuellement un courant secondaire riche en méthane.

11. Procédé selon l'une quelconque des revendications précédentes, **caractérisé en ce qu'**une troisième fraction (198) de la partie prélevée (190) est détendue, avant d'être directement réchauffée dans au moins une parmi la région additionnelle (54) d'échange thermique, la région aval (52) d'échange thermique, la région intermédiaire (50) d'échange thermique et la région amont (48) d'échange thermique.

12. Procédé selon l'une quelconque des revendications précédentes, **caractérisé en ce qu'**il comporte les étapes suivantes :
- séparation du courant de gaz craqué brut (100) en une première fraction (102) de gaz craqué brut et en une deuxième fraction (104) de gaz craqué brut ;
- refroidissement amont et condensation partielle de la première fraction (102) de gaz craqué brut dans la région amont (48) d'échange thermique ;
- refroidissement de la deuxième fraction (104) de gaz craqué brut dans un échangeur amont de rebouillage (64), par échange thermique avec un courant amont de rebouillage issu de la colonne amont (62), puis refroidissement de la deuxième fraction (104) de gaz craqué brut dans un échangeur intermédiaire de rebouillage (70) par échange thermique avec un courant intermédiaire de rebouillage issu de la colonne intermédiaire (68) ;
- formation du courant de gaz craqué brut partiellement condensé par mélange de la première fraction (104) de gaz craqué brut refroidie et de la deuxième fraction (106) de gaz craqué brut refroidie.

13. Procédé selon l'une quelconque des revendications précédentes, **caractérisé en ce que** la température du gaz craqué brut partiellement condensé avant son introduction dans le séparateur amont (42) est inférieure à - 25°C, **en ce que** la température du courant amont de gaz craqué partiellement condensé (122) avant son introduction dans le séparateur intermédiaire (44A) est inférieure à - 60°C, **en ce que** la température du courant intermédiaire partiellement condensé (160) avant son introduction dans l'ensemble aval de séparation (80) est inférieure à- 115°C.

14. Installation (24) de traitement d'un courant de gaz craqué (22) issu d'une installation (20) de pyrolyse d'hydrocarbures, du type comprenant :
- des moyens de refroidissement amont et de condensation partielle d'un courant de gaz craqué brut (100) comprenant au moins une région amont (48) d'échange thermique ;
- des moyens de séparation du courant de gaz craqué brut partiellement condensé comprenant au moins un séparateur amont (42) pour récupérer un liquide amont (106) et un courant amont (108) de gaz craqué ;
- une colonne amont (62) de déméthanisation et des moyens d'introduction du liquide amont (106) dans la colonne amont (62) pour récupérer en tête de la colonne amont (62), un courant amont de tête (114) riche en méthane et, en pied de la colonne amont, un premier courant liquide (112) riche en hydrocarbures en C₂⁺ ;
- des moyens de refroidissement intermédiaire et de condensation partielle du courant amont de gaz craqué (108) comprenant au moins une région intermédiaire (50) d'échange thermique ;
- des moyens de séparation du courant amont de gaz craqué partiellement condensé comprenant au moins un séparateur intermédiaire (44A, 44B) pour récupérer au moins un liquide intermédiaire (124, 136) et un courant intermédiaire (138) de gaz craqué ;
- une colonne intermédiaire (68) de déméthanisation et des moyens d'introduction du ou de chaque liquide intermédiaire (124, 140) dans la colonne intermédiaire (68) pour récupérer, en tête de la colonne intermédiaire (68), un courant intermédiaire de tête (146), et en pied de la colonne intermédiaire (68), un deuxième courant liquide (144) riche en hydrocarbures en C₂⁺ ;
- des moyens d'introduction d'au moins une partie du courant amont de tête (114) issu de la colonne amont (62) dans la colonne intermédiaire (68) ;
- des moyens de refroidissement aval et de condensation partielle du courant intermédiaire de gaz craqué (138) comprenant au moins une région aval (52) d'échange thermique ;
- des moyens de séparation du courant intermédiaire de gaz craqué partiellement condensé (160) comprenant un ensemble aval de séparation (80) pour récupérer un liquide aval (162) et un courant aval de gaz traité (170) ;
- des moyens d'introduction du liquide aval (162) dans la colonne intermédiaire de déméthanisation (68) ;
**caractérisée en ce que** l'installation comprend :
➢ des moyens de prélèvement d'une partie d'un liquide intermédiaire (136) issu d'un séparateur intermédiaire (44A, 44B) et des moyens de refroidissement de la partie prélevée (190) comprenant une région additionnelle (54) d'échange thermique ;
➢ des moyens de détente d'au moins une première fraction (194) de refroidissement obtenue à partir de la partie prélevée (190) et des moyens de mise en relation d'échange thermique de la première fraction de refroidissement détendue avec le courant intermédiaire de tête (146) comprenant un premier échangeur thermique de tête (74) pour condenser au moins partiellement le courant intermédiaire de tête (146) ;
➢ des moyens de séparation du courant intermédiaire de tête partiellement condensé comprenant un premier séparateur (76) de reflux pour former un courant liquide (148) de reflux introduit dans la colonne intermédiaire (68) et un premier courant gazeux de combustible (150) ;
➢ des moyens de réchauffage du premier courant gazeux de combustible (150) comprenant des moyens de passage dans au moins une région parmi la région additionnelle (54) d'échange thermique, la région aval (52) d'échange thermique, la région intermédiaire (50) d'échange thermique, et la région amont (48) d'échange thermique.

15. Installation (24) selon la revendication 14, **caractérisée en ce qu'**elle comprend :
- des moyens de formation du courant de gaz craqué brut (100) par compression d'un gaz craqué (22) issu de l'installation de pyrolyse (20) comportant au moins un appareil de compression (38, 40) ;
- des moyens de réchauffage de la première fraction de refroidissement (194) en aval du premier échangeur thermique de tête (74) comprenant des moyens de passage dans au moins une parmi la région additionnelle (54) d'échange thermique, la région aval (52) d'échange thermique, la région intermédiaire (50) d'échange thermique et la région amont (48) d'échange thermique ; et
- des moyens d'introduction de la première fraction de refroidissement réchauffée dans le gaz craqué (22) en amont ou au sein de l'appareil de compression (38, 40).

16. Installation (24) selon l'une quelconque des revendications 14 ou 15, **caractérisé en ce que** l'ensemble aval de séparation comporte une colonne (80) d'absorption d'éthylène, l'installation (24) comprenant :
- des moyens d'introduction du courant intermédiaire de gaz craqué partiellement condensé (160) dans la colonne d'absorption d'éthylène (80),
- des moyens de récupération d'un courant additionnel (166) de tête gazeux issu de la colonne d'absorption d'éthylène (80) ;
- des moyens de détente d'une deuxième fraction de refroidissement (196) obtenue à partir de la partie prélevée (190) ; et
- des moyens de mise en relation d'échange thermique de la deuxième fraction de refroidissement détendue avec le courant additionnel de tête (166) comprenant un deuxième échangeur thermique de tête (82) pour condenser au moins partiellement le courant additionnel de tête (166) ;
- des moyens de séparation du courant additionnel de tête partiellement condensé comprenant un deuxième séparateur de reflux (84) pour former un deuxième courant liquide de reflux (168) introduit dans la colonne d'absorption d'éthylène (80) et un courant gazeux traité (170).

17. Installation selon l'une quelconque des revendications 14 à 16, **caractérisée en ce qu'**elle comprend :
- des moyens de formation du courant de gaz craqué brut (100) par compression d'un gaz craqué (22) issu de l'installation de pyrolyse (20) comportant au moins un appareil de compression (38, 40) ;
- des moyens de réchauffage de la deuxième fraction de refroidissement (196), en aval du deuxième échangeur thermique de tête (82), comprenant des moyens de passage dans au moins une parmi la région additionnelle (54) d'échange thermique, la région aval (52) d'échange thermique, la région intermédiaire (50) d'échange thermique, et la région amont (48) d'échange thermique ;
- des moyens d'introduction de la deuxième fraction de refroidissement réchauffée dans le gaz craqué (22) issu de l'installation de pyrolyse (20), en amont ou au sein de l'appareil de compression (38, 40).

## Patentansprüche

1. Verfahren zur Behandlung eines gecrackten Gasstroms (22) aus einer Kohlenwasserstoff-Pyrolyse-Anlage (20) von der Art, welche die folgenden Schritte umfasst:
- oberstromiges Abkühlen und partielles Kondensieren eines gecrackten Rohgasstroms (100) in mindestens einer oberstromigen Wärmetauschregion (48),
- Trennen des teilweise kondensierten gecrackten Rohgasstroms in mindestens einem oberstromigen Separator (42), um eine oberstromige Flüssigkeit (106) und einen oberstromigen gecrackten Gasstrom (108) rückzugewinnen,
- Einleiten der oberstromigen Flüssigkeit (106) in eine oberstromige Entmethanisierungssäule (62), um am Kopf der oberstromigen Säule (62) einen methanreichen oberstromigen Kopfstrom (114) und am Fuß der oberstromigen Säule einen ersten, C₂⁺-kohlenwasserstoffreichen Flüssigkeitsstrom (112) rückzugewinnen,
- zwischenzeitliches Abkühlen und partielles Kondensieren des oberstromigen gecrackten Gasstroms (108) in mindestens einer Wärmetausch-Übergangsregion (50),
- Trennen des teilweise kondensierten gecrackten oberstromigen Gasstroms in mindestens einem Übergangsseparator (44A, 44B), um mindestens eine Übergangsflüssigkeit (124, 136) und einen gecrackten Übergangsgasstrom (138) rückzugewinnen,
- Einleiten der oder jeder Übergangsflüssigkeit (124, 140) in eine Übergangs-Entmethanisierungssäule (68), um am Kopf der Übergangssäule (68) einen Übergangskopfstrom (146) und am Fuß der Übergangssäule (68) einen zweiten, C₂⁺-kohlenwasserstoffreichen Flüssigkeitsstrom (144) rückzugewinnen,
- Einleiten mindestens eines Teils des oberstromigen Kopfstroms (114) aus der oberstromigen Säule (62) in die Übergangssäule (68),
- unterstromiges Abkühlen und partielles Kondensieren des gecrackten Übergangsgasstroms (138) in mindestens eine unterstromige Wärmetauschregion (52),
- Trennen des teilweise kondensierten gecrackten Übergangsgasstroms (160) in einer unterstromigen Trennungsanordnung (80), um eine unterstromige Flüssigkeit (162) und einen unterstromigen behandelten gasförmigen Strom (170) rückzugewinnen,
- Einleiten der unterstromigen Flüssigkeit (162) in die Entmethanisierungs-Übergangssäule (68),
**dadurch gekennzeichnet, dass** das Verfahren die folgenden Schritte umfasst:
- Entnehmen eines Teils einer Übergangsflüssigkeit (136) aus einem Übergangsseparator (44A, 44B) und Abkühlen des entnommenen Teils (190) in einer zusätzlichen Wärmetauschregion (54),
- Entspannen mindestens einer ersten, aus dem entnommenen Teil (190) gewonnenen Abkühlungsfraktion (194) und Versetzen der ersten entspannten Abkühlungsfraktion mit dem Übergangskopfstrom (146) in einem ersten Kopfwärmetauscher (74) in Wärmetauschbeziehung, um den Übergangskopfstrom (146) mindestens teilweise zu kondensieren,
- Trennen des teilweise kondensierten Übergangskopfstroms in einem ersten Rückflusstrenner (76), um einen flüssigen Rückflussstrom (148), der durch Gravitationsfließen in die Übergangssäule (68) gelangt, und einen ersten gasförmigen Brennstoffstrom (150) zu bilden,
- Entspannen und Erwärmen des ersten gasförmigen Brennstoffstroms (150) durch Durchgang in mindestens einer aus der zusätzlichen Wärmetauschregion (54), der unterstromigen Wärmetauschregion (52), der Wärmetausch-Übergangsregion (50) und/oder der oberstromigen Wärmetauschregion (48) ausgewählten Region.

2. Verfahren nach Anspruch 1, **dadurch gekennzeichnet, dass** die Übergangssäule (68) einen integrierten Wärmetauscher (69) aufweist, wobei die oder jede Übergangsflüssigkeit (124, 140) unter dem integrierten Wärmetauscher (69) und die unterstromige Flüssigkeit (162) über dem integrierten Wärmetauschers (69) eingeführt werden.

3. Verfahren nach Anspruch 1 oder 2, **dadurch gekennzeichnet, dass** es einen Schritt des Bildens des gecrackten Rohgasstroms (100) durch Kompression eines gecrackten Gases (22) aus der Pyrolyseanlage (20) in mindestens einem Kompressionsapparat umfasst, wobei das Verfahren die folgenden Schritte aufweist:
- Erwärmen der ersten Abkühlungsfraktion (194) im ersten Kopfwärmetauscher (74) in mindestens einer aus der zusätzlichen Wärmetauschregion (54), der unterstromigen Wärmetauschregion (52), der Wärmetausch-Übergangsregion (50) und/oder der oberstromigen Wärmetauschregion (48) ausgewählten Region und
- Einleiten der erwärmten ersten Abkühlungsfraktion in das gecrackte Gas (22) oberstromig zum oder in den Kompressionsapparat (38, 40).

4. Verfahren nach einem der vorangehenden Ansprüche, **dadurch gekennzeichnet, dass** der erste gasförmige Brennstoffstrom (150) aus dem ersten Rückflusstrenner (76) in einer ersten Turbine zur dynamischen Entspannung (88) entspannt und dann in mindestens einer aus der zusätzlichen Wärmetauschregion (54), der unterstromigen Wärmetauschregion (52), der Wärmetausch-Übergangsregion (50) und/oder der oberstromigen Wärmetauschregion (48) ausgewählten Region erwärmt wird, wobei das Verfahren einen Rekompressionsschritt des erwärmten ersten Brennstoffstroms (154) in mindestens einem ersten Kompressor (90) umfasst, der mit der ersten Turbine zur dynamischen Entspannung (88) gekoppelt ist.

5. Verfahren nach einem der vorangehenden Ansprüche, **dadurch gekennzeichnet, dass** der erste gasförmige Brennstoffstrom (150) aus dem ersten Rückflusstrenner (76) in einem Ventil statischer Entspannung entspannt wird.

6. Verfahren nach einem der vorangehenden Ansprüche, **dadurch gekennzeichnet, dass** die unterstromige Trennungsanordnung eine Ehtylenabsorptionssäule (80) aufweist, wobei das Verfahren die folgenden Schritte aufweist:
- Einleiten des teilweise kondensierten gecrackten Übergangsgasstroms (160) in die Ethylenabsorptionssäule (80),
- Rückgewinnen eines zusätzlichen gasförmigen Kopfstroms (166) aus der Ethylenabsorptionssäule (80),
- Entspannen einer zweiten Abkühlungsfraktion (196), die aus dem aus der Übergangsflüssigkeit entnommenen Teil (190) gewonnen wurde und
- Versetzen der zweiten entspannten Abkühlungsfraktion mit dem zusätzlichen Kopfstrom (166) in einem zweiten Kopfwärmetauscher (82) in Wärmetauschbeziehung, um den zusätzlichen Kopfstrom (166) mindestens teilweise zu kondensieren,
- Einleiten des teilweise kondensierten zusätzlichen Kopfstroms in einen zweiten Rückflusstrenner (84), um einen zweiten flüssigen Rückflussstrom (168), der durch Gravitationsfließen in die Ethylenabsorptionssäule (80) gelangt, und einen behandelten gasförmigen Strom (170) zu bilden.

7. Verfahren nach Anspruch 6, **dadurch gekennzeichnet, dass** es die folgenden Schritte umfasst:
- Bilden des gecrackten Rohgasstroms (100) durch Kompression eines gecrackten Gases (22) aus der Pyrolyseanlage (20) in einem Kompressionsapparat (38, 40),
- Erwärmen der zweiten Abkühlungsfraktion (190) nach dem zweiten Kopfwärmetauscher (82) in mindestens einer aus der zusätzlichen Wärmetauschregion (54), der unterstromigen Wärmetauschregion (52), der Wärmetausch-Übergangsregion (50) und/oder der oberstromigen Wärmetauschregion (48) ausgewählten Region,
- Einleiten der erwärmten zweiten Abkühlungsfraktion in das gecrackte Gas (22) aus der Pyrolyseanlage (20) oberstromig zum oder in den Kompressionsapparat (38, 40).

8. Verfahren nach einem der Ansprüche 6 oder 7, **dadurch gekennzeichnet, dass** es die folgenden Schritte umfasst:
- Entspannen mindestens eines ersten Teils (172) des behandelten gasförmigen Stroms (170) in mindestens einer zweiten Turbine zur dynamischen Entspannung (92),
- Erwärmen nach Entspannen des zweiten Teils (174) des behandelten gasförmigen Stroms (170) in mindestens einer aus der zusätzlichen Wärmetauschregion (54), der unterstromigen Wärmetauschregion (52), der Wärmetausch-Übergangsregion (50) und/oder der oberstromigen Wärmetauschregion (48) ausgewählten Region,
- Komprimieren des erwärmten ersten Teils (172) in mindestens einem zweiten Kompressor (94), der mit der zweiten Turbine zur dynamischen Entspannung (92) gekoppelt ist.

9. Verfahren nach einem der Ansprüche 6 oder 7, **dadurch gekennzeichnet, dass** es einen Entspannungsschritt mindestens eines ersten Teils (172) des behandelten gasförmigen Stroms (170) in einem Ventil statischer Entspannung umfasst.

10. Verfahren nach einem der Ansprüche 6 bis 9, **dadurch gekennzeichnet, dass** mindestens ein zweiter Teil (178) des behandelten gasförmigen Stroms (170) in eine Wasserstoffreinigungseinheit (96) eingeleitet wird, um einen mit wasserstoffreichen Strom (180) und einen Hilfsstrom (182) brennbaren Gases und eventuell einen methanreichen sekundären Strom zu produzieren.

11. Verfahren nach einem der vorangehenden Ansprüche, **dadurch gekennzeichnet, dass** eine dritte Fraktion (198) des entnommenen Teils (190) entspannt wird, bevor sie in mindestens einer aus der zusätzlichen Wärmetauschregion (54), der unterstromigen Wärmetauschregion (52), der Wärmetausch-Übergangsregion (50) und/oder der oberstromigen Wärmetauschregion (48) ausgewählten Region direkt erwärmt wird.

12. Verfahren nach einem der vorangehenden Ansprüche, **dadurch gekennzeichnet, dass** es die folgenden Schritte umfasst:
- Trennen des gecrackten Rohgasstroms (100) in eine erste gecrackte Rohgasfraktion (102) und in eine zweite gecrackte Rohgasfraktion(104),
- oberstromiges Abkühlen und partielles Kondensieren der ersten gecrackten Rohgasfraktion (102) in der oberstromigen Wärmetauschregion (48),
- Abkühlen der zweiten gecrackten Rohgasfraktion (104) in einem oberstromigen Siedetauscher (64) durch thermischen Austausch mit einem oberstromigen Siedestrom aus der oberstromigen Säule (62), danach Abkühlen der zweiten gecrackten Rohgasfraktion (104) in einem Übergangssiedetauscher (70) durch thermischen Austausch mit einem Übergangssiedestrom aus der Übergangssäule (68),
- Bilden des teilweise kondensierten gecrackten Rohgasstroms durch Mischen der ersten abgekühlten gecrackten Rohgasfraktion (104) und der zweiten abgekühlten gecrackten Rohgasfraktion (106).

13. Verfahren nach einem der vorangehenden Ansprüche, **dadurch gekennzeichnet, dass** die Temperatur des teilweise kondensierten gecrackten Rohgases vor seinem Einleiten in den oberstromigen Separator (42) unter -25 °C liegt, dass die Temperatur des teilweise kondensierten gecrackten oberstromigen Gasstroms (122) vor seinem Einleiten in den Übergangsseparator (44A) unter -60 °C liegt und dass die Temperatur des teilweise kondensierten Übergangsstroms (160) vor seinem Einleiten in die unterstromige Trennungsanordnung (80) unter -115 °C liegt.

14. Anlage (24) zur Behandlung eines gecrackten Gasstroms (22) aus einer Kohlenwasserstoff-Pyrolyse-Anlage (20) der Bauart, die umfasst:
- Mittel zum oberstromigen Abkühlen und partiellen Kondensieren eines gecrackten Rohgasstroms (100), die mindestens eine oberstromige Wärmetauschregion (48) umfassen,
- Mittel zum Trennen des teilweise kondensierten gecrackten Rohgasstroms, die mindestens einen oberstromigen Separator (42) zur Rückgewinnung einer oberstromigen Flüssigkeit (106) und eines oberstromigen gecrackten Gasstroms (108) umfassen,
- eine oberstromige Entmethanisierungssäule (62) und Mittel zum Einleiten der oberstromigen Flüssigkeit (106) in die oberstromige Säule (62), um am Kopf der oberstromigen Säule (62) einen oberstromigen methanreichen Kopfstrom (114) und am Fuß der oberstromigen Säule einen ersten C₂⁺-kohlenwasserstoffreichen Flüssigkeitsstrom (112) rückzugewinnen,
- Mittel zum zwischenzeitlichen Abkühlen und partiellen Kondensieren des oberstromigen gecrackten Gasstroms (108), die mindestens eine Wärmetausch-Übergangsregion (50) umfassen,
- Mittel zum Trennen des oberstromigen, teilweise kondensierten gecrackten Gasstroms, die mindestens einen Übergangsseparator (44A, 44B) zur Rückgewinnung von mindestens einer Übergangsflüssigkeit (124, 136) und einem gecrackten Übergangsgasstrom (138) umfassen,
- eine Übergangs-Entmethanisierungssäule (68) und Mittel zum Einleiten der oder jeder Übergangsflüssigkeit (124, 140) in die Übergangssäule (68), um am Kopf der Übergangssäule (68) einen Übergangskopfstrom (146) und am Fuß der Übergangssäule (68) einen zweiten C₂⁺-kohlenwasserstoffreichen Flüssigkeitsstrom (144) rückzugewinnen,
- Mittel zum Einleiten von mindestens einem Teil des oberstromigen Kopfstroms (114) aus der oberstromigen Säule (62) in die Übergangssäule (68),
- Mittel zum unterstromigen Abkühlen und partiellen Kondensieren des gecrackten Übergangsgasstroms (138), die mindestens eine unterstromige Wärmetauschregion (52) umfassen,
- Mittel zum Trennen des teilweise kondensierten gecrackten Übergangsgasstroms (160), die eine unterstromige Trennungsanordnung (80) zur Rückgewinnung einer unterstromigen Flüssigkeit (162) und eines unterstromigen behandelten gasförmigen Stroms (170) umfassen,
- Mittel zum Einleiten der unterstromigen Flüssigkeit (162) in die Entmethanisierungs-Übergangssäule (68),
**dadurch gekennzeichnet, dass** die Anlage umfasst:
➢ Mittel zum Entnehmen eines Teils einer Übergangsflüssigkeit (136) aus einem Übergangsseparator (44A, 44B) und Mittel zum Abkühlen des entnommenen Teils (190), die eine zusätzliche Wärmetauschregion (54) umfassen,
➢ Mittel zum Entspannen mindestens einer ersten, aus dem entnommenen Teil (190) gewonnenen Abkühlungsfraktion (194) und Mittel zum Versetzen der ersten entspannten Abkühlungsfraktion in Wärmetauschbeziehung mit dem Übergangskopfstrom (146), die einen ersten Kopfwärmetauscher (74) umfassen, um den Übergangskopfstrom (146) mindestens teilweise zu kondensieren,
➢ Mittel zum Trennen des teilweise kondensierten Übergangskopfstroms, die einen ersten Rückflusstrenner (76) umfassen, um einen flüssigen Rückflussstrom (148), der in die Übergangssäule (68) gelangt, und einen ersten gasförmigen Brennstoffstrom (150) zu bilden,
➢ Mittel zum Erwärmen des ersten gasförmigen Brennstoffstroms (150), die Durchgangsmittel in mindestens einer aus der zusätzlichen (54) Wärmetauschregion, der unterstromigen Wärmetauschregion (52), der Wärmetausch-Übergangsregion (50) und/oder der oberstromigen Wärmetauschregion (48) ausgewählten Region umfassen.

15. Anlage (24) nach Anspruch 14, **dadurch gekennzeichnet, dass** sie umfasst:
- Mittel zum Bilden des gecrackten Rohgasstroms (100) durch Kompression eines gecrackten Gases (22) aus der Pyrolyseanlage (20), die mindestens einen Kompressionsapparat (38, 40) umfassen,
- Mittel zum Erwärmen der ersten Abkühlungsfraktion (194) nach dem ersten Kopfwärmetauscher (74), die Durchgangsmittel in mindestens einer aus der zusätzlichen Wärmetauschregion (54), der unterstromigen Wärmetauschregion (52), der Wärmetausch-Übergangsregion (50) und/oder der oberstromigen Wärmetauschregion (48) ausgewählten Region umfassen, und
- Mittel zum Einleiten der erwärmten ersten Abkühlungsfraktion in das gecrackte Gas (22) oberstromig zum oder in den Kompressionsapparat (38, 40).

16. Anlage (24) nach einem der Ansprüche 14 oder 15, **dadurch gekennzeichnet, dass** die unterstromige Trennungsanordnung eine Ethylenabsorptionssäule (80) aufweist, wobei die Anlage (24) umfasst:
- Mittel zum Einleiten des teilweise kondensierten gecrackten Übergangsgasstroms (160) in die Ethylenabsorptionssäule (80),
- Mittel zum Rückgewinnen eines zusätzlichen gasförmigen Kopfstroms (166) aus der Ethylenabsorptionssäule (80),
- Mittel zum Entspannen einer zweiten Abkühlungsfraktion (196), die aus dem entnommenen Teil (190) gewonnen wurde und
- Mittel zum Versetzen der zweiten entspannten Abkühlungsfraktion in Wärmetauschbeziehung mit dem zusätzlichen Kopfstrom (166), die einen zweiten Kopfwärmetauscher (82) umfassen, um den zusätzlichen Kopfstrom (166) mindestens teilweise zu kondensieren,
- Mittel zum Trennen des teilweise kondensierten zusätzlichen Kopfstroms, die einen zweiten Rückflusstrenner (84) umfassen, um einen zweiten flüssigen Rückflussstrom (168), der in die Ethylenabsorptionssäule (80) gelangt, und einen behandelten gasförmigen Strom (170) zu bilden.

17. Anlage nach einem der Ansprüche 14 bis 16, **dadurch gekennzeichnet, dass** sie umfasst:
- Mittel zum Bilden des gecrackten Rohgasstroms (100) durch Kompression eines gecrackten Gases (22) aus der Pyrolyseanlage (20), die mindestens einen Kompressionsapparat (38, 40) umfassen,
- Mittel zum Erwärmen der zweiten Abkühlungsfraktion (196) nach dem zweiten Kopfwärmetauscher (82), die Durchgangsmittel in mindestens einer aus der der zusätzlichen Wärmetauschregion (54), der unterstromigen Wärmetauschregion (52), der Wärmetausch-Übergangsregion (50) und/oder der oberstromigen Wärmetauschregion (48) ausgewählten Region umfassen,
- Mittel zum Einleiten der erwärmten zweiten Abkühlungsfraktion in das gecrackte Gas (22) aus der Pyrolyseanlage (20) oberstromig zum oder in den Kompressionsapparat (38, 40).

## Claims

1. A method for treating a cracked gas stream (22) stemming from a hydrocarbon pyrolysis installation (20), of the type comprising the following steps:
- upstream cooling and partial condensation of a crude cracked gas stream (100) in at least one upstream heat exchange region (48);
- separating the partly condensed crude gas stream in at least one upstream separator (42) in order to recover an upstream liquid (106) and an upstream cracked gas stream (108);
- introducing the upstream liquid (106) into an upstream demethanization column (62) for recovering at the head of the upstream column (62), an upstream head stream (114) rich in methane and, at the bottom of the upstream column, a first liquid stream (112) rich in C₂⁺ hydrocarbons;
- intermediate cooling and partial condensation of the upstream cracked gas stream (108) in at least one intermediate heat exchange region (50);
- separating the partly condensed upstream cracked gas stream in at least one intermediate separator (44A, 44B) for recovering at least one intermediate liquid (124, 136) and one intermediate cracked gas stream (138);
- introducing the or each intermediate liquid (124, 140) into an intermediate demethanization column (68) in order to recover at the head of the intermediate column (68), an intermediate head stream (146), and at the bottom of the intermediate column (68), a second liquid stream (144) rich in C₂⁺ hydrocarbons;
- introducing at least one portion of the upstream head stream (114) from the upstream column (62) into the intermediate column (68);
- downstream cooling and partial condensation of the intermediate cracked gas stream (138) in at least one downstream heat exchange region (52);
- separating the intermediate partly condensed cracked gas stream (160) in a downstream separation assembly (80) for recovering a downstream liquid (162) and a downstream treated gas stream (170);
- introducing the downstream liquid (162) into the intermediate demethanization column (68).
**characterized in that** the method comprises the following steps:
- sampling a portion of an intermediate liquid (136) from an intermediate separator (44A, 44B) and cooling of the sampled portion (190) in an additional heat exchange region (54);
- expansion of at least one first cooling fraction (194) obtained from the sample portion (190) and putting the first expansed cooling fraction in a heat exchange relationship with the intermediate head stream (146) in a first head heat exchanger (74) for at least partly condensing the intermediate head stream (146);
- separating the intermediate partly condensed head stream in a first reflux separator (76) in order to form a liquid reflux stream (148) introduced into the intermediate column (68) by gravity flow, and a first combustible gas stream (150);
- expansion and heating-up of the first combustible gas stream (150) by having it pass in at least one region among the additional heat exchange region (54), the downstream heat exchange region (52), the intermediate heat exchange region (50), and the upstream heat exchange region (48).

2. The method according to claim 1, **characterized in that** the intermediate column (68) includes an integrated heat exchanger (69), said or each intermediate liquid (124, 140) being introduced below the integrated heat exchanger (69), the downstream liquid (162) being introduced above the integrated heat exchanger (69).

3. The method according to claim 1 or 2, **characterized in that** it comprises a step for forming the crude cracked gas stream (100) by compression of a cracked gas (22) stemming from the pyrolysis installation (20) in at least one compression apparatus, the method comprising the following steps:
- heating up the first cooling fraction (194) in the first head heat exchanger (74) in at least one region among the additional heat exchange region (54), the downstream heat exchange region (52), the intermediate heat exchange region (50) and the upstream heat exchange region (48); and
- introducing the first cooling fraction heated up in the cracked gas (22) upstream from or within the compression apparatus (38, 40).

4. The method according to any of the preceding claims, **characterized in that** the first combustible gas stream (150) from the first reflux separator (76) is expanded in a first dynamic expansion turbine (88), and then heated up in at least one region among the additional heat exchange region (54), the downstream heat exchange region (52), the intermediate heat exchange region (50) and the upstream heat exchange region (48), the method comprising a step for compressing again the first heated-up combustible stream (154) in at least one first compressor (90) coupled with the first dynamic expansion turbine (88).

5. The method according to any of the preceding claims, **characterized in that** the first combustible gas stream (150) from the first reflux separator (76) is expanded in a static expansion valve.

6. The method according to any of the preceding claims, **characterized in that** the downstream separation assembly includes an ethylene absorption column (80), the method comprising the following steps:
- introducing the intermediate partly condensed cracked gas stream (160) into the ethylene absorption column (80),
- recovering an additional head gas stream (166) from the ethylene absorption column (80);
- expanding a second cooling fraction (196) obtained from the portion (190) sampled in the intermediate liquid, and
- putting the second expansed cooling fraction into a heat exchange relationship with the additional head stream (166) in a second head heat exchanger (82) in order to at least partly condense the additional head stream (166);
- introducing the partly condensed additional head stream into a second reflux separator (84) in order to form a second liquid reflux stream (168) introduced into the ethylene absorption column (80) by gravity flow and a treated gas stream (170).

7. The method according to claim 6, **characterized in that** it comprises the following steps:
- forming the crude cracked gas stream (100) by compression of a cracked gas (22) from the pyrolysis installation (20) in a compression apparatus (38, 40):
- heating up the second cooling fraction (190), downstream from the second head heat exchanger (82), in at least one region among the additional heat exchange region (54), the downstream heat exchange region (52), the intermediate heat exchange region (50) and the upstream heat exchange region (48);
- introducing the second heated-up cooling fraction into the cracked gas (22) from the pyrolysis installation (20), upstream from or within the compression apparatus (38, 40).

8. The method according to any of claims 6 or 7, **characterized in that** it comprises the following steps:
- expanding at least one first portion (172) of the treated gas stream (170) in at least one second dynamic expansion turbine (92),
- heating up after expansion the first portion (174) of the treated gas stream (170) in at least one region among the additional heat exchange region (54), the downstream heat exchange region (52), the intermediate heat exchange region (50) and the upstream heat exchange region (48);
- compression of the heated-up first portion (172) in at least one second compressor (94) coupled with the second dynamic expansion (92).

9. The method according to any of claims 6 or 7, **characterized in that** it comprises a step for expanding at least one first portion (172) of the treated gas stream (170) in a static expansion valve.

10. The method according to any of claims 6 to 9, **characterized in that** at least one second portion (178) of the treated gas stream (170) is introduced into a hydrogen purification unit (96) for producing a hydrogen-rich stream (180) and an auxiliary combustible gas stream (182), and optionally a secondary stream rich in methane.

11. The method according to any of the preceding claims, **characterized in that** a third fraction (198) of the sampled portion (190) is expanded before being directly heated up in at least one region among the additional heat exchange region (54), the downstream heat exchange region (52), the intermediate heat exchange region (50) and the upstream heat exchange region (48).

12. The method according to any of the preceding claims, **characterized in that** it includes the following steps:
- separating the crude cracked gas stream (100) into a first crude cracked gas fraction (102) and into a second crude cracked gas fraction (104);
- upstream cooling and partial condensation of the first crude cracked gas fraction (102) in the upstream heat exchange region (48);
- cooling the second crude cracked gas fraction (104) in an upstream reboiling exchanger (64), by heat exchange with an upstream reboiling stream from the upstream column (62), and then cooling the second crude cracked gas fraction (104) in an intermediate reboiling exchanger (70) by heat exchange with an intermediate reboiling stream from the intermediate column (68);
- forming the partly condensed crude cracked gas stream by mixing the first cooled crude cracked gas fraction (104) and the second cooled crude cracked gas fraction (106).

13. The method according to any of the preceding claims, **characterized in that** the temperature of the partly condensed crude cracked gas before its introduction into the upstream separator (42) is below - 25°C, **in that** the temperature of the partly condensed upstream cracked gas stream (122) before its introduction into the intermediate separator (44A) is below - 60°C, **in that** the temperature of the partly condensed intermediate stream (160) before its introduction into the downstream separation assembly (80) is below - 115°C.

14. An installation (24) for treating a cracked gas stream (22) from a hydrocarbon pyrolysis installation (20) of the type comprising:
- means for upstream cooling and partial condensation of a crude cracked gas stream (100) comprising at least one upstream heat exchange region (48);
- means for separating the partly condensed crude cracked gas stream comprising at least one upstream separator (42) for recovering an upstream liquid (106) and an upstream cracked gas stream (108);
- an upstream demethanization column (62), and means for introducing the upstream liquid (106) into the upstream column (62) in order to recover at the head of the upstream column (62), an upstream head stream (114) rich in methane and at the bottom of the upstream column, a first liquid stream (112) rich in C₂⁺ hydrocarbons;
- means for intermediate cooling and partial compensation of the upstream cracked gas stream (108) comprising at least one intermediate heat exchange region (50);
- means for separating the upstream partly condensed cracked gas stream comprising at least one intermediate separator (44A, 44B) in order to recover at least one intermediate liquid (124, 136), and an intermediate cracked gas stream (138);
- an intermediate demethanization column (68) and means for introducing the or each intermediate liquid (124, 140) into the intermediate column (68) in order to recover at the head of the intermediate column (68), an intermediate head stream (146), and at the bottom of the intermediate column (68), a second liquid stream (144) rich in C₂⁺ hydrocarbons;
- means for introducing at least one portion of the upstream head stream (114) from the upstream column (62) into the intermediate column (68);
- means for downstream cooling and partial condensation of the intermediate cracked gas stream (138) comprising at least one downstream heat exchange region (52);
- means for separating the intermediate partly condensed cracked gas stream (160) comprising a downstream separation assembly (80) in order to recover a downstream liquid (162) and a downstream treated gas stream (170);
- means for introducing the downstream liquid (162) into the intermediate demethanization column (68);
**characterized in that** the installation comprises:
➢ means for sampling a portion of an intermediate liquid (136) from an intermediate separator (44A, 44B), and means for cooling the sampled portion (190) comprising an additional heat exchange region (54);
➢ means for expanding at least one first cooling fraction (194) obtained from the sampled portion (190) and means for putting the first expanded cooling fraction in a heat exchange relationship with the intermediate head stream (146) comprising a first head heat exchanger (74) for at least partly condensing the intermediate head stream (146);
➢ means for separating the intermediate partly condensed head stream comprising a first reflux separator (76) in order to form a reflux liquid stream (148) introduced into the intermediate column (68) and a first combustible gas stream (150);
➢ means for heating up the first combustible gas stream (150) comprising means for passing into at least one region among the additional heat exchange region (54), the downstream heat exchange region (52), the intermediate heat exchange region (50) and the upstream heat exchange region (48).

15. The installation (24) according to claim 14, **characterized in that** it comprises:
- means for forming the crude cracked gas stream (100) by compression of a cracked gas (22) from the pyrolysis installation (20) including at least one compression apparatus (38, 40);
- means for heating up the first cooling fraction (194) downstream from the first head heat exchanger (74) comprising means for passing into at least one region among the additional heat exchange region (54), the downstream heat exchange region (52), the intermediate heat exchange region (50) and the upstream heat exchange region (48); and
- means for introducing the heated-up first cooling fraction into the cracked gas (22) upstream from or within the compression apparatus (38, 40).

16. The installation (24) according to any of claims 14 or 15, **characterized in that** the downstream separation assembly includes an ethylene absorption column (80), the installation (24) comprising:
- means for introducing the intermediate cracked gas stream (160) into the ethylene absorption column (80),
- means for recovering an additional head gas stream (166) from the ethylene absorption column (80);
- means for expanding a second cooling fraction (196) obtained from the sampled portion (190); and
- means for putting the second expanded cooling fraction in a heat exchange relationship with the additional head stream (166) comprising a second head heat exchanger (82) for at least partly condensing the additional head stream (166);
- means for separating the additional partly condensed head stream comprising a second reflux separator (84) for forming a second liquid reflux stream (168) introduced into the ethylene absorption column (80) and a treated gas stream (170).

17. The installation according to any of claims 14 to 16, **characterized in that** it comprises:
- means for forming the crude cracked gas stream (100) by compression of a cracked gas (22) sfrom the pyrolysis installation (20) including at least one compression apparatus (38, 40);
- means for heating up the second cooling fraction (196), downstream from the second head heat exchanger (82), comprising means for passing into at least one region among the additional heat exchange region (54), the downstream heat exchange region (52), the intermediate heat exchange region (50) and the upstream heat exchange region (48);
- means for introducing the second heated-up cooling fraction into the cracked gas (22) from the pyrolysis installation (20), upstream from or within the compression apparatus (38, 40).
